# EUROPEAN PATENT SPECIFICATION

(11) **EP 1 389 092 B1**
(45) Date of publication and mention of the grant of the patent: **15.11.2006**
(21) Application number: 02731767.6
(22) Date of filing: 10.05.2002
(51) Int. Cl.: A61K 9/20, A61K 45/06, A61K 31/485, A61P 25/36

(54) **ABUSE-RESISTANT OPIOID DOSAGE FORM**
OPIOID ENTHALTENDE ARZNEIFORM GEGEN MISSBRAUCH
FORME DE DOSAGE D'OPIOIDES EMPECHANT LA CONSOMMATION ABUSIVE

(30) Priority: 11.05.2001 US 290438 P
(43) Date of publication of application: 18.02.2004
(73) Proprietor: Endo Pharmaceuticals Inc., Chadds Ford, PA 19317 (US)
(72) Inventor: KAO, Huai-Hung, Syosset, NY 11791 (US); ZENG, Yadi, Fort Lee, NJ 07024 (US); HOWARD-SPARKS, Michelle, Ridgewood, NY 11385 (US); JIM, Fai, Franklin Square, Ny 11010 (US)
(74) Representative: Buckley, Guy Julian
(86) International application number: PCT/US2002/015021
(87) International publication number: WO 2002/092059

(56) References cited:
- WO-A-01/58447
- WO-A-99/11250
- WO-A-99/32120

## Description

### FIELD OF THE INVENTION

The present invention relates to abuse resistant opioid compositions.

### BACKGROUND OF THE RELATED ART

Morphine, a classic opioid, has been known as a very powerful analgesic compound for many years. Its potential as a target of abuse has been known for almost as long. Morphine and other opioids and derivatives are used in the pharmaceutical industry as narcotic analgesics, hypnotics, sedatives, anti-diarrheals, anti-spasmodics, and anti-tussives. Most often, they are used as powerful analgesics. Opioids are well known to have addictive effects. Despite the potential for addiction and abuse, opioids are widely used due to their superior, powerful analgesic properties. Such opioids include codeine, dihydrocodeine, hydrocodone, hydromorphone, levorphanol, meperidine, buprenorphine, fentanyl, fentanyl derivatives, dipipanone, heroin, tramadol, etorphine, dihydroetorphine, butorphanol, methadone, morphine, oxycodone, oxymorphone, and propoxyphene. In the past, abuse of opioids has been generally limited to illicit drugs made in illegal laboratories. Abuse of pharmaceutical opioids was quite limited. Accordingly, action by makers of pharmaceutical opioids would, in the past, have little or no effect on illegal abuse of opioids.

Recently, however, the trend has been changing. Abuse of pharmaceutical opioids has been increasing. This is especially true in the case of extended release opioid dosage forms. Extended release opioid dosage forms are intended for decreased frequency of dosing. Therefore, each tablet must contain the amount of opioid which would be contained in several immediate release tablets. This results in the production of dosage forms having substantially increased amounts of opioid. A single extended release tablet can provide much more opioid to the potential abuser than low dose, immediate release dosage forms. This results in stronger feeling of euphoria, or "high" from controlled release tablets than an abuser would get from an immediate release tablet. This makes such tablets more desirable for an abuser.

Previous attempts at abuse resistant opioid compositions for oral administration have included an opioid which has substantial activity orally as well as activity when administered by injection, in combination with an opioid antagonist which is less effective orally than by injection. This helps prevent abuse involving crushing and dissolving the composition followed by injection. Most prescription opioid analgesic pharmaceutical compositions are tablets designed for oral administration. Therefore opioid antagonists which have very low oral bioavailability, have little action when taken orally at parenterally effective doses. Therefore, the antagonist has little effect when the tablet is taken as intended but greatly enhanced effect if the tablet is abused parenterally.

Such opioid antagonists have substantially increased effect when taken directly into the blood stream. Thus, abusing the opioid by crushing the tablet, dissolving it, and injecting or snorting (intranasal administration), would cause the antagonist to have its full effect, essentially blocking the opioid receptors, preventing the abuser from receiving an opioid effect, and inducing withdrawal in opioid-dependent individuals.

Furthermore, in the past, tablets were relatively low-dosage, and contained low levels of opioid compared to the extended release tablets in use today, and many more tablets were needed for abusers Therefore oral abuse was more difficult and less common. With the increase in oral abuse of extended release opioid compositions, it would be beneficial to develop a tablet that would make oral abuse more difficult, less desirable, and aversive for opioid abusers. One patent application which describes attempts to solve the problem of abuse of controlled release of opioids is PCT patent application publication WO 01/58451 to Euroceltique, S.A. This publication discusses a tamper-resistant oral opioid agonist formulation having an opioid agonist in releasable form, and a sequestered opioid antagonist that is substantially not released when the dosage form is administered intact. The ratio of the amount of opioid antagonist released from the dosage form after tampering to the amount of the antagonist released from the intact dosage form is 4:1 or greater. However while this may help deter abuse involving the crushing of a tablet there is still a need for abuse resistant opioid formulations. The present invention is directed to such a tablet.

### SUMMARY OF THE INVENTION

The present invention is as set out in the accompanying claims.

The present invention pertains to a controlled release pharmaceutical dosage form comprising an opioid agonist and an opioid antagonist contained in a single tablet. The antagonist is in both immediate and controlled release forms. A portion of the antagonist can be in the same matrix as the agonist and in a matrix separate from that of the agonist.

The present invention also pertains to a controlled release pharmaceutical dosage form comprising an opioid agonist in a matrix and an opioid antagonist in a matrix separate from the opioid agonist matrix as well as in a coating on the tablet. The separate matrix for the antagonist allows independent release rates to be achieved for the opioid agonist and antagonist, while the antagonist in the coating or immediate release layer allows release of some antagonist immediately when the tablet is taken. The antagonist can be released very slowly, or it can be partially contained, and partially released when the tablet is taken orally. Crushing the tablet allows full release of the opioid antagonist, preventing or discouraging abuse. Further because not all of the opioid antagonist is sequestered, dissolving the tablet will also release sufficient opioid antagonist to discourage parenteral abuse. However, normal administration will not release sufficient antagonist to affect the analgesic properties of the agonist.

### DETAILED DESCRIPTION OF THE PREFERRED EMBODIMENT

The present invention relies on the principle that certain substances are undesirable when an opioid is abused orally or parenterally. One group of such substances, opioid antagonists, reverses and blocks the opioid response. Opioid antagonists can block a response regardless of how administered, but some are much more potent when administered parenterally than orally. Thus, if any antagonist is introduced in sufficient quantities with an opioid to an intended abuser, the antagonist will block the desired euphoric effect and may induce withdrawal, depending on the dose given. If such an antagonist is introduced into a pharmaceutical tablet, once abusers determine that the tablet will not produce a euphoric effect, and may induce withdrawal, abusers may cease to abuse the tablet as it will not help them achieve their goal of obtaining a euphoric effect. If the tablet induces withdrawal in an addict, the addict will eschew the tablet, as induction of withdrawal is a particularly disturbing event. Induced withdrawal for an opioid addict can present itself with symptoms including nausea, vomiting, cold sweats, chills, anxiety, paranoia, aches, cramps, muscle spasms, and a host of other uncomfortable symptoms. A tablet which induces withdrawal would be undesirable to an addict. Therefore, the production of such a tablet or other dosage form will curb abuse. Of course, the tablet must, at the same time, be effective for a patient taking the tablet or other dosage form for its therapeutic analgesic effect. Although reference is made herein to "tablets," one skilled in the art will recognize that the present invention can be applied equally to capsules or other dosage forms.

The tablet of the present invention is an analgesic opioid pharmaceutical dosage form for oral administration. The dosage form is, in some ways, similar to those already produced and used for relief of moderate to severe pain in individuals. Often, the currently-marketed tablets are used for pain relief in cancer patients and other patients experiencing severe pain. However, the tablet of the present invention differs from prior art tablets by including a mechanism for deterring abuse. This mechanism centers around opioid antagonists included in the tablet. The antagonists can be in a matrix which provides a reduced release rate, or in a matrix which provides essentially little or no release of the agent when the tablet is taken orally. Thus the antagonist is sequestered. Additional antagonist is added for immediate release with the opioid. This additional antagonist may be the same as or different from the first agonist.

One problem with prior art tablets, even those having a sequestered antagonist, is that careful dissolution of the tablet without crushing (such as by leaving the tablet in water overnight) will extract opioid without antagonist, allowing abuse. Addicts are surprisingly resourceful at devising methods of abuse. Therefore, this route to abuse should be closed.

Accordingly, the present invention includes opioid agonist and two different portions of opioid antagonist. The first matrix contains opioid antagonist and is prepared in such a manner so as to sequester and slow or prevent completely the release of the antagonist. The first matrix can be in the form of microparticles, dispersed evenly throughout the second matrix, or it can take another form. The second matrix generally forms the bulk of the tablet and includes the opioid agonist. The second matrix is a standard matrix for a tablet of the type desired (either controlled release for long-acting tablets, or immediate release for normal (4 hour) tablets). Where the first matrix is in another form, it can, for instance, form a solid core of the tablet with the second matrix surrounding it, or it may form a layer, in a multi-layer tablet. Where the first matrix is in the form of small particles, or where it forms the core of the tablet, a coating may be used to slow the release of the opioid antagonist from the first matrix. In either case, it is important that crushing the tablet will release the opioid in the first matrix, whereas dissolving the tablet slowly (as occurs when the tablet is taken by a patient) will not. Further antagonist is provided in immediate release form to prevent careful dissolution and abuse of the tablet.

As mentioned above, the tablet includes a second dose of opioid antagonist in an immediate release form. This antagonist is released when a patient takes the tablet. Preferably, this antagonist is induced in the tablet at a low level, such that taking the tablet in a normal fashion will not antagonize the analgesic property of the opioid. However, if an abuser dissolves the tablet slowly and administers the resulting supernatant liquid parenterally, the antagonist will antagonize the opioid and may induce withdrawal in dependent individuals. This operates to deter the careful dissolution and abuse of the tablet. The immediate release antagonist can be contained either in a coating or in a separate immediate release matrix layer. The antagonist used in the immediate release form can be any suitable antagonist, including naloxone, naltrexone, nalorphine, diprenorphine, levallorphan, pentazocine, metazocine, cyclazocine, etazocine, N -cyclopropylmethyl - 7,8 - dihydro - 14 - hydroxynormorphinone, or 21 -cyclopropyl z, - (1 - hydroxy - 1 - methylethyl) - 6,14 - endo - ethano -tetrahydrooripavine (or diphenorphine).

In a preferred embodiment, a different opioid antagonist is used in the first matrix from that in the third matrix or coating. Specifically, it is preferred to use naloxone in the third matrix or coating. Naloxone has a very high oral:parenteral ratio. Naloxone exhibits very low bioavailability when administered orally, yet exhibits high bioavailability and effectiveness when administered parenterally. Therefore, including naloxone in the third matrix or coating will allow a patient using the tablet to receive naloxone orally. Yet due to its low bioavailability, the naloxone will have little or no effect on the patient. However, should an abuser dissolve the tablet slowly and administer the resulting solution parenterally, the naloxone will have full antagonistic activity. The term "parenteral" as used herein is intended to include any administration where the opioid is not absorbed through the digestive track. This includes, without limitation, intravenous, sublingual and intra-nasal administration.

In this embodiment, it is preferred to use an opioid antagonist other than naloxone in the first matrix. Preferred antagonists for the first matrix include naltrexone, nalmefene, levallorphan, cyclazacine, or mixtures thereof. These antagonists exhibit good antagonistic effect when administered orally. Therefore, the antagonist will produce undesirable effects upon an abuser who chews or crushes the tablet and administers it orally. Alternatively, additional naloxone can be included to overcome low oral bioavailability, but this will have an unintended increased effect if administered parenterally.

The third matrix should contain sufficient antagonist to prevent abuse. This amount may vary with tablet strength, but generally, at least about 0.2 mg, preferably at least about 1 mg, more preferably at least 2 mg, most preferably at least about 10 mg antagonist should be used in the third matrix of the tablet. The third matrix should include sufficient antagonist to prevent parenteral abuse, but not enough to cause an effect on the oral user.

The first, sequestering, matrix containing the antagonist in the tablet of the invention substantially prevents release of the antagonist under normal circumstances (i.e. when the intact tablet is taken orally). Therefore, the tablet may be loaded with a sufficient dosage of the antagonist that, despite the reduced oral efficacy of the antagonist, should the tablet be crushed or chewed and taken orally, the dose of antagonist will be sufficient to prevent the euphoric opioid effect and may also induce withdrawal. Thus, the tablet of the present invention will also prevent oral abuse of orally administered controlled release tablets, which are becoming more commonly abused. With oral abuse, abusers chew or crush a controlled release opioid tablet to convert the tablet to immediate release in order to obtain a euphoria or high. In this circumstance, or if the tablet is dissolved and injected, the opioid antagonist will prevent the abuser from receiving a euphoric high and may also cause withdrawal in opioid-dependent individuals, thus, deterring abuse. Thus the tablet of the present invention should prevent abuse by administration of the tablet in any altered form, whether crushed or dissolved, and whether swallowed, snorted, or injected. Furthermore, this tablet is compatible with other abuse-deterring agents or systems.

The tablet of the present invention can be used with a wide range of opioids. Specifically, it is most preferable to use the tablet of the present invention with opioids having a high potential for abuse. Opioid agonists used in the present invention can be any agonist in general use as an analgesic, including but not limited to codeine, dihydrocodeine, hydrocodone, hydromorphone, levorphanol, meperidine, buprenorphine, fentanyl, fentanyl derivatives, dipipanone, heroin, tramadol, etorphine, dihydroetorphine, butorphanol, methadone, morphine, oxycodone, oxymorphone, and propoxyphene and pharmaceutically acceptable salts thereof. Specifically, any addictive opioid in an oral tablet form is the target of the present invention. Most particularly, controlled release oxycodone has recently been the target of abuse and would therefore make a good candidate for use in the present invention. However, while controlled release tablets have recently been a particular problem, the tablet of the present invention may be used for immediate release tablets as well as those in a controlled release format.

In the tablet of the present invention, the opioid antagonist is contained in a separate matrix from the opioid agonist. That separate matrix can be formed in many different ways. One appropriate configuration is a uniform controlled release matrix with the opioid antagonist dispersed therein. That controlled release matrix is formulated and granulated into very small granules. These granules are then incorporated into the main matrix of the tablet. In this way, the antagonist is contained in a separate controlled release matrix that forms part of the entire tablet. The granules can also be coated to further sequester the antagonist prior to incorporation into the tablet. Upon ingestion, the low, orally-ineffective dose of opioid antagonist would dissolve, along with the (the matrix may/may not dissolve)the opioid agonist. This dissolution releases the opioid agonist and the granules containing the orally-effective dose of opioid antagonist in a reduced release or non-release matrix. The antagonist-containing granules then pass through and out of the body, releasing only minimal therapeutically ineffective amounts of opioid antagonist, or not at all.

Another possible configuration for the tablet of the present invention incorporates the opioid antagonist into an immediate release matrix. The matrix can then be granulated and coated with a non-release coating, such as an acrylic polymer. The granules are then incorporated into either an immediate release or a controlled release opioid tablet. The tablet is then coated with antagonist. Upon administration, the tablet releases antagonist and opioid at a predetermined rate, but the coated granules releases no antagonist. Rather, the granules pass through the intestines and are then eliminated from the patient. In this way, the coated granules act as an excipient and, under normal circumstances, have no pharmacological effect whatsoever. Any suitable controlled or immediate release matrix can be used to sequester the opioid antagonist provided that the proper non-release coating is used along and that the matrix and agent are compatible.

Alternatively, a reduced release rate granule could be formed using an immediate release matrix with a reduced release rate coating over the formed granules. Although the description of the invention describes a "non-release" matrix in one embodiment, it is possible that some leakage of opioid antagonist may occur where "non-release" is specified. This is acceptable as long as the release rate is very low (lower than necessary to have a significant pharmalogical effect). This is particularly significant where the antagonist has high oral bioavailability and can affect the therapeutic action of the tablet if released. Thus, the definition of non-release, as used herein, should include any reduced release matrix which allows less than 30 percent of an opioid antagonist to be released over a 12-hour period under normal conditions of oral administration. Of course, none of the "non-release" matrices described herein are intended to fully encapsulate the opioid antagonist or other agents so as to prevent release when the tablet is crushed or dissolved. Furthermore, a suitable non-release coating can be formed by using several known coatings together on a granulated matrix containing opioid antagonist. For instance, the agonist-containing granules can be covered with a coating which allows for release of material only at a pH below 5 (or 3), which is then covered by a coating which allows release of material only at above a pH of 5 (or 7 or even 9). In that way, when the tablet is ingested, the outer coating will prevent release of agonist while the granules reside in the stomach, and the inner coating will prevent release of material once the tablet has passed through the stomach into the intestines, where the pH rises sufficiently to dissolve the outer coating. One skilled in the art would be able to formulate a suitable matrix for use in the tablet of the present invention.

The amount of antagonist used in the tablet will vary with the amount of opioid agonist used (i.e., with the tablet strength), the therapeutic dose of the antagonist, and the route of administration to be prevented. In the case of injection or intranasal administration, only about 0.2-0.4 mg naloxone is needed to antagonize the opioid effect, to induce abstinence in dependent individuals, and to prevent abuse. However, because of the reduced efficacy of naloxone when taken orally, substantially greater amounts are needed to prevent oral abuse when naloxone is used as the sequestered antagonist. Accordingly, there should be at least about 0.1 mg, preferably at least 1.0 mg, more preferably at least about 5.0 mg, and most preferably at least about 20 mg per tablet to prevent oral abuse. Small amounts of antagonists with greater oral bioavailability can be used. The amount of naloxone in each tablet will vary with tablet strength, both because a greater amount of opioid in the tablet can require a larger amount of antagonist to counteract, but also because, with higher strength tablets, abusers may divide the tablets into several smaller doses, and it would be most desirable to ensure that each dose has sufficient antagonist to prevent abuse. Thus, a 160 mg oxycodone tablet should have more opioid antagonist than a 10 or 20 mg oxycodone tablet. The ratio of opioid:opioid antagonist may vary from 1:3 to 2:1 because the naloxone is used in a reduced-rate release matrix, or in a non-release matrix, allowing large amounts of naloxone to be incorporated into a tablet. Thus, a tablet could incorporate 100 mg of naloxone or more in a non-release format.

Regarding opioid antagonists, the foregoing has been described with respect to naloxone, but the present invention is intended to encompass the use of any appropriate known opioid antagonist, including, but not limited to: naloxone, naltrexone, nalorphine, diprenorphine, levallorphan, pentazocine, metazocine, cyclazocine, etazocine, N -cyclopropylmethyl - 7,8 - dihydro - 14 - hydroxynormorphinone, or 21 -cyclopropyl z, - (1 - hydroxy - 1 - methylethyl) - 6,14 - endo - ethano -tetrahydrooripavine (or diphenorphine) and the pharmaceutically acceptable acid addition salts thereof. Preferably, the antagonist is one which, like naloxone, has substantially greater effectiveness when administered by injection than when administered orally.

In a further embodiment of the present invention, the opioid antagonist is not encapsulated and dispersed in the body of the tablet, but rather is contained in the center of the tablet and surrounded with a controlled release matrix. The surrounding matrix contains an opioid agonist. When the tablet is swallowed whole, the surrounding matrix releases opioid at a controlled rate. The rate is selected such that the tablet is eliminated from the body prior to release of the antagonist in the center of the tablet. Alternatively, additional layers may be used to further control release of the opioid. For example, the outermost level may release a large dose of opioid, to provide fast pain relief, followed by a slower release to provide continued relief over time. The layers could alternatively release opioid agonist and opioid antagonist. For instance, the tablet could be layered to produce a slow release of opioid followed by a fast spike of antagonist, followed by a slow release of opioid and then a fast spike of antagonist. In this manner, the slow release of opioid will first occupy receptors and the spike of antagonist will occur in insufficient quantity and will undergo faster metabolism, and thus will not affect the action of the opioid. If the tablet is crushed, a large bolus of antagonist would be released, interfering with the action of the agonist, deterring future abuse.

The following examples, while not intended to limit the invention in any way, are illustrative of the present invention.

### EXAMPLE 1

**FORMULATION A: 10 mg Oxycodone HCl / 20 mg Naloxone HCl**

| **Naloxone NR Granules A** | |
|---|---|
| **Ingredient** | **Amount/Unit (mg)** |
| Naloxone HCl | 10.00 |
| Microcrystalline Cellulose | 18.66 |
| Eudragit RS30D | 22.93 |
| Surelease | 6.91 |
| Sub-Total | 58.50 |
| | |

| **Tablet A - NR Layer** | |
|---|---|
| Naloxone NR Granules A | 58.50 |
| Oxycodone HCl | 10.00 |
| Microcrystalline Cellulose | 30.88 |
| Eudragit RSPO | 28.98 |
| Sodium Lauryl Sulfate | 2.86 |
| Magnesium Hydroxide | 0.21 |
| Povidone | 5.36 |
| Cab-O-Sil | 1.43 |
| Stearic Acid | 0.89 |
| Magnesium Stearate | 0.89 |
| | |

| **Naloxone IR Coating** | |
|---|---|
| Naloxone HCl | 10.00 |
| Opadry Pink | 15.00 |
| Water | N/A |
| Total | 165.00 |

### PROCESS:

### Naloxone NR Granules A

1. Mix Naloxone and Microcrystalline Cellulose.
2. Spray Eudragit RS30D (30% suspension) to the powder in fluid bed dryer. Dry at 60°C.
3. Spray Surrelease (15% suspension) to the granules in fluid bed dryer. Dry at 60°C.

### Tablet A

1. Mix all excipients of the NR layer except Stearic Acid and Magnesium Stearate.
2. Mix Stearic Acid and Magnesium Stearate with granules.
3. Compress to tablet.

### Immediate Release Naloxone Coating

1. Dissolve Naloxone HCl in Opadry Pink suspension (15%).
2. Spray to Tablet A.

### DISSOLUTION

Dissolution was conducted according to USP XXIV Apparatus II (Paddle Method.) at 75 rpm using 0.1N HCl as dissolution medium. The bath temperature is set at 37.5°C. The HPLC parameters are set as follows: Column - Inertsil ODS 3, 50 mm x 4.6 mm, 3 µm particle size. Mobile phase: 80% 30 mM sodium hexanesulfonate pH 3.0 +/- 1, 20% acetonitrile. Injection volume is 75 µL. Column temperature is 35 °C, Flow rate is set at 1.0 mL/min. Wavelength is set at 225 nm. Run time is 5.5 minutes.

### RESULTS AND DISCUSSION

| **Formulation A** | | |
|---|---|---|
| | **Tablet A not Crushed** | |
| **Time** | **% Oxycodone Dissolved** | **% Naloxone Dissolved** |
| 0 | 0.0 | 0.0 |
| 1 | 34.7 | 72.3 |
| 2 | 49.4 | 73.1 |
| 3 | 59.5 | 74.3 |
| 4 | 66.7 | 75.8 |
| 8 | 85.9 | 82.9 |
| 12 | 97.2 | 90.5 |

### EXAMPLE 2

**FORMULATION B: 10 mg Oxycodone HCl /10 mg Naloxone HCl**

| **Naloxone NR Granules B** | |
|---|---|
| **Ingredient** | **Amount/Unit (mg)** |
| Naloxone HCl | 7.0 |
| Dicalcium Phosphate | 52.0 |
| Eudragit L30D-55 | 20.7 |
| Eudragit RS30D | 12.4 |
| Sub-Total | 92.1 |
| | |

| **Tablet B-NR Layer** | |
|---|---|
| Naloxone NR Granules B | 92.1 |
| Oxycodone HCI | 10.0 |
| Microcrystalline Cellulose | 22.5 |
| Eudragit RSPO | 119.3 |
| Povidone 29/32 | 13.3 |
| Cab-O-Sil | 5.3 |
| Magnesium Stearate | 2.7 |
| Total | 265.0 |
| | |

| **Tablet B-IR Layer** | |
|---|---|
| Naloxone HCI | 3.0 |
| Microcrystalline Cellulose | 58.1 |
| Povidone 29/32 | 2.0 |
| Cab-O-Sil | 1.3 |
| Magnesium Stearate | 0.7 |
| Total | 65.0 |
| | |
| Overall Tablet B Weight | 330.0 |

### PROCESS:

### Naloxone NR Granules B

1. Mix Naloxone and Dicalcium Phosphate.
2. Spray Eudragit L30D-55 (30% suspension) to the powder in fluid bed dryer. Dry at 60°C.
3. Spray Eudragit R30D (30% suspension) to the granules in fluid bed dryer. Dry at 60°C.

### Tablet B-NR Layer

1. Mix all excipients of the NR layer except Magnesium Stearate.
2. Mix Magnesium Stearate with granules.
3. Compress to tablet.

### Tablet B-IR/NR Bi-Layers

1. Mix all excipients of the IR layer except Magnesium Stearate.
2. Add and mix Magnesium Stearate to the IR blend.
3. Compress the immediate release layer on top of Tablet B-NR layer to form bi-layer tablets.
4. Cure the tablet at 80°C for 12 hours.

### DISSOLUTION

Dissolution was conducted according to USP XXIV Apparatus I (Basket Method.) at 100 rpm using Simulated Gastric Fluid at pH 1.2 (0.1N HCl with Sodium Chloride) without enzyme in the first hour and Simulated Intestine Fluid at pH 6.8 (10mM Phosphate Buffer without enzyme) from 2 to 12 hours as dissolution medium. The bath temperature is set at 37.5°C. The HPLC parameters is set as follows: Column - Inertsil ODS 3, 50 mm x 4.6 mm, 3 µm particle size. Mobile phase: 80% 30 mM sodium hexanesulfonate pH 3.0 +/- 1, 20% acetonitrile. Injection volume is 75 µL. Column temperature is 35 °C, Flow rate is set at 1.0 mL/min. Wavelength is set at 225 nm. Run time is 5.5 minutes.

### RESULTS AND DISCUSSION

| **Formulation B** | | |
|---|---|---|
| | **Tablet B not Crushed** | |
| **Time** | **% Oxycodone Dissolved** | **% Naloxone Dissolved** |
| 0 | 0.0 | 0.0 |
| 1 | 33.4 | 49.7 |
| 2 | 48.6 | 60.7 |
| 3 | 57.7 | 67.3 |
| 4 | 63.9 | 72.0 |
| 8 | 78.9 | 83.2 |
| 10 | 82.9 | 86.2 |

## Claims

1. An abuse resistant oral pharmaceutical dosage form comprising:
a first matrix including a first opioid antagonist wherein the matrix provides a reduced release rate or no release of the antagonist;
a second matrix including an opioid agonist; and
a coating including a second opioid antagonist in an immediate release form.

2. An abuse resistant oral pharmaceutical dosage form comprising:
a first matrix including a first opioid antagonist wherein the matrix provides a reduced release rate or no release of the antagonist;
a second matrix including an opioid agonist; and
a third matrix including a second opioid antagonist in an immediate release form.

3. The abuse resistant oral pharmaceutical dosage form of Claims 1 or 2 wherein said dosage form is a tablet which, when intact, is adapted to release at least 30% of the total opioid antagonist in the first hour based on dissolution according to USP XXIV Apparatus I, basket method at 100 rpm using 0.1 N HCl as dissolution medium at 37.5°C (preferably at least 40% in the first hour and not more than 65% in 12 hours).

4. The abuse resistant oral pharmaceutical dosage form of Claims 1-3 wherein when said dosage form is a tablet it is adapted to release, when crushed, at least 75% of the total opioid antagonist in the first hour based on dissolution according to USP XXIV Apparatus I, Basket method at 100 rpm using 0.1 N HCl as dissolution medium at 37.5°C.

5. The abuse resistant oral pharmaceutical dosage form of Claims 1-4 wherein said opioid agonist is selected from the group consisting of codeine, dihydrocodeine, hydrocodone, hydromorphone, levorphanol, meperidine, buprenorphine, fentanyl, fentanyl derivatives, dipipanone, heroin, tramadol, etorphine, dihydroetorphine, butorphanol, oxycodone, oxymorphone, methadone, morphine, and propoxyphene and pharmaceutically acceptable salts thereof.

6. The abuse resistant oral pharmaceutical dosage form of Claims 1-5 wherein at least one of said opioid antagonists is selected from the group consisting of naloxone, naltrexone, nalorphine, diprenorphine, levallorphan, pentazocine, metazocine, cyclazocine, etazocine, N -cyclopropylmethyl - 7,8 - dihydro - 14 - hydroxynormorphinone, and 21 -cyclopropyl z, - (1 - hydroxy - 1 - methylethyl) - 6,14 - endo - ethano -tetrahydrooripavine (or diphenorphine).

7. The abuse resistant oral pharmaceutical dosage form of Claims 1-6 wherein said first and said second opioid antagonist are the same or different.

8. The abuse resistant oral pharmaceutical dosage form of Claim 7 wherein said first opioid antagonist is naltrexone and said second opioid antagonist is naloxone.

9. The abuse resistant oral pharmaceutical dosage form of Claims 1-8 wherein when said dosage form is a tablet it is adapted to release at least about 50% of the total antagonist in the first hour.

10. The abuse resistant oral pharmaceutical dosage form of claims 1-9 wherein said first matrix is dispersed in said second matrix.

11. The abuse resistant oral pharmaceutical dosage form of claims 1-10, wherein said first matrix is coated to prevent release of said first opioid antagonist

12. The abuse resistant oral pharmaceutical dosage form of Claims 1 and 3-11 wherein said coating includes an additional opioid antagonist.

## Patentansprüche

1. Eine missbrauchssichere Arzneiform zur oralen Verabreichung, die Folgendes umfasst:
eine erste Matrix, die einen ersten Opioidantagonisten beinhaltet, wobei die Matrix für eine niedrigere Freisetzungsrate des Antagonisten sorgt oder die Freisetzung verhindert;
eine zweite Matrix, die einen Opioidagonisten beinhaltet; und
eine Beschichtung, die einen zweiten Opioidantagonisten in einer Form beinhaltet, die eine sofortige Freisetzung ermöglicht.

2. Eine missbrauchssichere Arzneiform zur oralen Verabreichung, die Folgendes umfasst:
eine erste Matrix, die einen ersten Opioidantagonisten beinhaltet, wobei die Matrix für eine niedrigere Freisetzungsrate des Antagonisten sorgt oder die Freisetzung verhindert;
eine zweite Matrix, die einen Opioidagonisten beinhaltet; und
eine dritte Matrix, die einen zweiten Opioidantagonisten in einer Form beinhaltet, die eine sofortige Freisetzung ermöglicht.

3. Die missbrauchssichere Arzneiform zur oralen Verabreichung aus den Ansprüchen 1 oder 2, wobei die Arzneiform eine Tablette ist, die, im intakten Zustand, basierend auf Auflösung gemäß USP XXIV Apparat I, Basket Methode bei 100 Upm unter Verwendung von 0,1 N HCl als Lösungsmittel bei 37,5°C in der ersten Stunde wenigstens 30 % des gesamten Opioidantagonisten (vorzugsweise wenigstens 40 % in der ersten Stunde und nicht mehr als 65 % in 12 Stunden) freisetzen kann.

4. Die missbrauchssichere Arzneiform zur oralen Verabreichung aus den Ansprüchen 1-3, wobei wenn die Arzneiform eine Tablette ist, diese im zerkleinerten Zustand in der ersten Stunde zumindest 75 % des gesamten Opioidantagonisten basierend auf Auflösung gemäß USP XXIV Apparat I, Korbmethode bei 100 Upm unter Verwendung von 0,1 N HCl als Lösungsmittel bei 37,5° C freisetzen kann.

5. Die missbrauchssichere Arzneiform zur oralen Verabreichung aus den Ansprüchen 1-4, wobei der Opioidantagonist aus der Gruppe bestehend aus Codein, Dihydrocodein, Hydrocodon, Hydromorphon, Levorphanol, Meperidin, Buprenorphin, Fentanyl, Fentanylderivaten, Dipipanon, Heroin, Tramadol, Etorphin, Dihydroetorphin, Butorphanol, Oxycodon, Oxymorphon, Methadon, Morphin und Propoxyphen und deren pharmazeutisch verträglichen Salzen ausgewählt ist.

6. Die missbrauchssichere Arzneiform zur oralen Verabreichung aus den Ansprüchen 1-5, wobei wenigstens einer der Opioidantagonisten aus der Gruppe bestehend aus Naloxon, Naltrexon, Nalorphin, Diprenorphin, Levallorphan, Pentazocin, Metazocin, Cyclazocin, Etazocin, N-Cyclopropylmethyl-7,8,-dihydro-14-hydroxynormorphinon und 21-Cyclopropyl-(1-hydroxy-1-methylethyl)-6,14-endo-ethano-tetrahydrooripavin (oder -diphenorphin) ausgewählt ist.

7. Die missbrauchssichere Arzneiform zur oralen Verabreichung aus den Ansprüchen 1-6, wobei der erste und der zweite Opioidantagonist einander entsprechen oder sich voneinander unterscheiden.

8. Die missbrauchssichere Arzneiform zur oralen Verabreichung aus Anspruch 7, wobei der erste Opioidantagonist Naltrexon und der zweite Opioidantagonist Naloxon ist.

9. Die missbrauchssichere Arzneiform zur oralen Verabreichung aus den Ansprüchen 1-8, wobei, wenn die Arzneiform eine Tablette ist, diese wenigstens 50% des gesamten Opioidantagonisten in der ersten Stunde freisetzen kann.

10. Die missbrauchssichere Arzneiform zur oralen Verabreichung aus den Ansprüchen 1-9, wobei die erste Matrix in der zweiten Matrix dispergiert ist.

11. Die missbrauchssichere Arzneiform zur oralen Verabreichung aus den Ansprüchen 1-10, wobei die erste Matrix beschichtet ist um eine Freisetzung des ersten Opioidantagonisten zu verhindern.

12. Die missbrauchssichere Arzneiform zur oralen Verabreichung aus den Ansprüchen 1 und 3-11, wobei die Beschichtung einen zusätzlichen Opioidantagonisten beinhaltet.

## Revendications

1. Formulation pharmaceutique orale pour lutter contre la toxicomanie comprenant :
une première matrice incluant un premier antagoniste opioïde dans laquelle la matrice n'offre qu'une libération réduite, voire nulle, de l'antagoniste :
une seconde matrice contenant un agoniste opioïde : et
un enrobage renfermant un deuxième antagoniste opioïde à libération immédiate.

2. Formulation pharmaceutique orale pour lutter contre la toxicomanie comprenant :
une première matrice contenant un premier antagoniste opioïde dans laquelle la matrice n'offre qu'une libération réduite, voire nulle, de l'antagoniste ;
une seconde matrice contenant un agoniste opioïde, et
une troisième matrice contenant un deuxième antagoniste opioïde à libération immédiate.

3. Formulation pharmaceutique orale de lutte contre la toxicomanie selon les revendications 1 ou 2, dans laquelle ladite formulation a la forme d'un comprimé qui, s'il est intact, est prévu pour libérer au moins 30% de l'antagoniste opioïde dans la première heure, basé sur le processus de dissolution selon le protocole USP XXIV Dispositif 1, méthode d'immersion au panier à 100 t/mn utilisant comme milieu de dissolution 0.1 NHCL à 37.5°C (de préférence au moins 40% dans la première heure et pas plus de 65% en douze heures).

4. Formulation pharmaceutique orale de lutte contre la toxicomanie selon les revendications 1 à 3 dans laquelle si le comprimé est écrasé, il est conçu pour libérer au moins 75% de l'antagoniste opioïde dans la première heure, basé sur le processus de dissolution selon le protocole USP XXIV Dispositif 1, méthode d'immersion au panier à 100 t/mn utilisant comme milieu de dissolution 0.1 NHCL à 37.5°C.

5. Formulation pharmaceutique orale de lutte contre la toxicomanie selon les revendications 1 à 4 dans laquelle ledit antagoniste opioïde est choisi parmi un groupe comprenant codéine, dihydrocodéine, hydrocodone, hydromorphone, levorphanol, mépéridine, buprénorphine, fentanyl et ses dérivés, dipinanone, héroïne, tramadol, étorphine, dihydroétorphine, butorphanol, méthadone, morphine, oxycodone, oxymorphone et propoxyphène et leurs sels reconnus dans la pharmacopée.

6. Formulation pharmaceutique orale de lutte contre la toxicomanie selon les revendications 1 à 5 dans laquelle au moins un desdits antagonistes opioïdes est sélectionné dans un groupe comprenant la naloxone, naltrexone, nalorphine, diprénorphine, levallorphan, pentazocine, métazocine, cyclazocine, étazocine, N-cyclopropylmethyl-7. [8-dihydro-14-hydroxynormorphinone,] et 21-cyclopropyle z, - (1-hydroxy-1-méthyléthyle)-6.14-endo-éthano-tétrahydrooripavine(ou diphénorphine).

7. Formulation pharmaceutique orale de lutte contre la toxicomanie selon les revendications 1 à 6 dans laquelle lesdits premier et deuxième antagonistes opioïdes sont semblables ou différents.

8. Formulation pharmaceutique orale de lutte contre la toxicomanie selon la revendications 7, dans laquelle ledit premier antagoniste opioïde est la naltrexone et ledit second antagoniste opioïde est la naloxone.

9. Formulation pharmaceutique orale de lutte contre la toxicomanie selon les revendications 1 à 8, dans laquelle lorsque ladite formulation est un comprimé, il est conçu pour libérer au moins 50% de l'antagoniste total durant la première heure.

10. Formulation pharmaceutique orale de lutte contre la toxicomanie selon les revendications 1 à 9, dans laquelle ladite première matrice est dispersée dans ladite seconde matrice.

11. Formulation pharmaceutique orale de lutte contre la toxicomanie selon les revendications 1 à 10, dans laquelle ladite première matrice est enrobée pour empêcher la libération dudit premier antagoniste opioïde.

12. Formulation pharmaceutique orale de lutte contre la toxicomanie selon les revendications 1 et 3 à 11. dans laquelle ledit enrobage contient un antagoniste opioïde supplémentaire.
